# EUROPEAN PATENT SPECIFICATION

(11) **EP 2 569 446 B1**
(45) Date of publication and mention of the grant of the patent: **11.07.2018**
(21) Application number: 11780925.1
(22) Date of filing: 12.05.2011
(51) Int. Cl.: C12Q 1/68

(54) **DIAGNOSTIC MARKERS FOR NEUROPSYCHIATRIC DISEASEN**
DIAGNOSTISCHE MARKER FÜR NEUROPSYCHIATRISCHE ERKRANKUNGEN
MARQUEURS DE DIAGNOSTIQUE POUR LES MALADIES NEUROPSYCHIATRIQUES

(30) Priority: 12.05.2010 US 395354 P
(43) Date of publication of application: 20.03.2013
(73) Proprietor: Schutzer, Steven E., Water Mill, NY 11976 (US)
(72) Inventor: Schutzer, Steven E., Water Mill, NY 11976 (US)
(74) Representative: Teuten, Andrew John
(86) International application number: PCT/US2011/000843
(87) International publication number: WO 2011/142827

(56) References cited:
- WO-A1-2010/103451
- WO-A2-2008/125651
- US-A1- 2008 274 456
- US-A1- 2009 124 542
- "Human Genome U95Av2", INTERNET CITATION, 2 October 2002 (2002-10-02), XP002215481, Retrieved from the Internet: URL:http://www.affymetrix.com [retrieved on 2002-10-02]
- "GeneChip Human Genome U133 Set", INTERNET CITATION, 26 February 2003 (2003-02-26), XP002232760, Retrieved from the Internet: URL:http://www.affymetrix.com/support/tech nical/datasheets/hgu133_datasheet.pdf [retrieved on 2003-02-26]
- CONSTANTINE L ET AL: "Use of genechip high-density oligonucleotide arrays for gene expression monitoring", LIFE SCIENCE NEWS, AMERSHAM LIFE SCIENCE, US, 1 January 1998 (1998-01-01), pages 11-14, XP002964122, ISSN: 0969-0190
- XU JING ET AL: "Characterization of Proteome of Human Cerebrospinal Fluid", INTERNATIONAL REVIEW OF NEUROBIOLOGY, ACADEMIC PRESS, LONDON, GB, vol. 73, 1 March 2009 (2009-03-01), pages 29-89, XP009176808, ISSN: 0074-7742, DOI: 10.1016/S0074-7742(06)73002-1
- STEVEN E. SCHUTZER ET AL: "Establishing the Proteome of Normal Human Cerebrospinal Fluid", PLOS ONE, vol. 5, no. 6, 1 January 2010 (2010-01-01) , page e10980, XP055106566, ISSN: 1932-6203, DOI: 10.1371/journal.pone.0010980
- T. LIU ET AL: "High Dynamic Range Characterization of the Trauma Patient Plasma Proteome", MOLECULAR & CELLULAR PROTEOMICS, vol. 5, no. 10, 29 May 2006 (2006-05-29), pages 1899-1913, XP055106552, ISSN: 1535-9476, DOI: 10.1074/mcp.M600068-MCP200
- ALEXANDRE ZOUGMAN ET AL: "Integrated Analysis of the Cerebrospinal Fluid Peptidome and Proteome", JOURNAL OF PROTEOME RESEARCH, vol. 7, no. 1, 1 January 2008 (2008-01-01) , pages 386-399, XP055106541, ISSN: 1535-3893, DOI: 10.1021/pr070501k
- DATABASE BIOSIS [Online] BIOSCIENCES INFORMATION SERVICE, PHILADELPHIA, PA, US; April 2009 (2009-04), RITHIDECH K N ET AL: "Protein expression profiles in pediatric multiple sclerosis: potential biomarkers", XP009176952, Database accession no. PREV200900274388
- HONG JIAN ET AL: "Gene expression profiling of relevant biomarkers for treatment evaluation in multiple sclerosis", JOURNAL OF NEUROIMMUNOLOGY, ELSEVIER SCIENCE PUBLISHERS BV, NL, vol. 152, no. 1-2, 1 July 2004 (2004-07-01), pages 126-139, XP002566863, ISSN: 0165-5728, DOI: 10.1016/J.JNEUROIM.2004.03.004
- SATOH ET AL.: 'The 14-3-3 protein epsilon isoform expressed in reactive astrocytes in demyelinating lesions of multiple sclerosis binds to vimentin and glial fibrillary acidic protein in cultured human astrocytes' AMERICAN JOURNAL OF PATHOLOGY vol. 165, no. 2, 31 August 2004, pages 577 - 592, XP007916328
- ZHANG ET AL.: 'Quantitative proteomic analysis of age-related changes in human cerebrospinal fluid' NEUROBIOL AGING vol. 26, no. 2, February 2005, pages 207 - 227, XP004670613
- NATELSON ET AL.: 'Spinal fluid abnormalities in patients with chronic fatigue syndrome' CLINICAL AND DIAGNOSTIC LABORATORY IMMUNOLOGY vol. 12, no. IS. 1., 31 January 2005, pages 52 - 55, XP055064558
- TUMANI ET AL.: 'Relevance of cerebrospinal fluid variables for earty diagnosis of neuroborteliosis' NEUROLOGY vol. 45, no. 9, September 1995, pages 1663 - 1670, XP055064562
- DOTEVALL ET AL.: 'Astroglial and neuronal proteins in cerebrospinal fluid as markers of CNS involvement in lyme neuroborreliosis' EUROPEAN J NEUROLOGY vol. 6, no. 2, March 1999, pages 169 - 178, XP055064564
- SCHUTZER ET AL.: 'Distinct Cerebrospinal Fluid Proteomes Differentiate Post-Treatment Lyme Disease from Chronic Fatigue Syndrome, art e17287' PLOS ONE vol. 6, no. IS. 2, 23 February 2011, pages 1 - 8, XP055065944

## Description

### FIELD OF THE INVENTION

The present invention relates to identifying biologic markers (biomarkers) that can be used for diagnosis of multiple sclerosis. In particular embodiments, the present invention relates to identifying biomarkers for diagnosis of multiple sclerosis. In a more particular aspect, the invention pertains to the identification of a biomarker signature that is diagnostic for multiple sclerosis,. Accordingly, the invention further relates to a method for diagnosing a person or other mammal with multiple sclerosis, or at risk of developing same. The biomarkers and diagnostic signatures described herein also identify metabolic/biochemical pathways as potential candidates for therapeutic targeting. The invention further relates to guidance pertaining to appropriate treatment of the person or mammal diagnosed with multiple sclerosis in accordance the methods described herein. Accurate diagnosis of multiple sclerosis, particularly with respect to diseases that are difficult to distinguish clinically, should reduce the duration and/or severity of the disease by ensuring that the patient is treated using an appropriate therapeutic regimen.

### BACKGROUND OF THE INVENTION

Knowledge of the entire protein content, the proteome, of normal human cerebrospinal fluid (CSF) would provide a critical standard to allow meaningful comparisons with and between neurologic and psychiatric disorders. CSF contains both cellular and soluble components providing insights into processes occurring in the central nervous system (CNS). As much as 30 to 40% of CSF is formed by the extracellular fluid of the brain and spinal cord. CSF contains both normal and disease specific components, and provides an accessible liquid window into the brain. In fact, recent data suggest CSF may provide more relevant evidence for initial or propagating pathology than the brain parenchyma itself in certain neuropsychiatric diseases [Ransohoff (2009) Nature 457: 155-156]. Comprehensive characterization of the normal CSF proteome, furthermore, facilitates identification of disease-specific markers [Ekegren et al. (2008) J Mass Spectrom 43: 559-571]. Knowledge of which proteins are present, absent, or of changed concentrations may lead to diagnostic, prognostic, or disease-activity biomarkers as well as provide insights into disease etiology and pathogenesis. An advantage of a full proteome analysis is the ability to identify not just one but a multitude of proteins at a single instance.

Multiple sclerosis, for example, is an excellent candidate disease for such an approach because it is often a progressive debilitating disease that is difficult to diagnose definitively, especially during the early stages of the disease. Multiple sclerosis is a common demyelinating disease of the central nervous system (CNS) that affects up to 0.1% of the Caucasian population of northern European descent. Multiple sclerosis is more common in women than men and generally begins between ages 20 and 40, but can develop at any age. Multiple sclerosis is generally viewed as an autoimmune syndrome directed against unidentified central nervous tissue antigens. The determination of susceptibility to multiple sclerosis development is complex and appears to be governed by both environmental and genetic factors. Some of the symptoms of multiple sclerosis are caused by damage to the myelin sheath, the protective covering that surrounds nerve cells. When this nerve covering is damaged, nerve impulses are slowed down or stopped. Ultimately, damage to the myelin sheath results in nerve damage. Multiple sclerosis is, moreover, commonly a progressive disease that leads to more severe neurologic dysfunction over time. Nerve damage may be caused by inflammation that occurs when the body's immune cells attack the nervous system. Repeated episodes of inflammation can occur along any area of the brain and spinal cord, which is why the disease is often referred to as one characterized by symptoms and signs over time and space.

Multiple sclerosis is difficult to diagnose because the progress, severity and specific symptoms of multiple sclerosis are quite variable and unpredictable. There are no laboratory tests, symptoms or physical findings that can singly determine if a person has multiple sclerosis. The differential diagnosis of multiple sclerosis is quite varied and includes metabolic, genetic, oncologic, immunologic, and infectious disease assessment. Other diseases that may need to be considered in the differential diagnoses, but should be considered depending on the clinical presentation, include: Acute disseminated encephalomyelitis, CNS vasculitis, Behçet disease, Sjögren syndrome, Sarcoid, neoplasms, CADASIL (cerebral autosomal dominant arteriopathy with subcortical infarcts and leukoencephalopathy), Migrainous ischemia, Cerebrovascular disease, Progressive multifocal leukoencephalopathy, Inherited white matter diseases, effects of radiation therapy or drugs, CNS lymphoma, Lyme disease, HTLV-1 infection, CNS lupus, Mitochondrial encephalopathies, Antiphospholipid antibody syndrome, cerebral emboli, Thrombocytopenic purpura, Progressive multifocal leukoencephalopathy, Mycoplasma encephalopathy, Vitamin B12 deficiency, Paraneoplastic syndromes, Psychiatric syndromes (Rolak LA, Fleming JO. The Neurologist 2007;13: 57-72). Over the last twenty years, tests such as magnetic resonance imaging (MRI), examination of CSF, and evoked response testing have played an increasingly important role in the diagnostic process. In 2005, revised McDonald criteria for multiple sclerosis were published (Polman et al. Diagnostic Criteria for Multiple Sclerosis: 2005 Revisions to the "McDonald" Criteria. Ann Neurol. (2005) 58:840-846 and Polman et al. Ann Neurol (2011) 69:292-302). In addition to the traditional diagnostic tools, the revised criteria provide specific guidelines for using findings of MRI, cerebrospinal fluid analysis and visual evoked potentials to support a diagnosis of multiple sclerosis. However, even with these revised criteria, diagnosis of multiple sclerosis is still challenging and frequently takes several months or even years.

Rendering a conclusive diagnosis of multiple sclerosis on an expedited basis would be of great benefit to patients in light of the potential for recurrence of attacks and progression of the disease. Drugs for the treatment of multiple sclerosis are now available which slow or prevent progression of the disease in many patients, and an early diagnosis would, therefore, allow early intervention and could significantly improve the quality of life for many multiple sclerosis patients.

It is therefore an object of the present invention to provide an efficient diagnosis system for neuropsychiatric disease in general, a particular example of which is multiple sclerosis, so as to provide biomarkers with which a clinician can diagnose or exclude a particular disease in a patient or other mammal. The same reasoning applies to Neurologic Lyme Disease and Chronic Fatigue syndrome.

Other features and advantages of the invention will be apparent from the detailed description, the drawings, and the claims.

### SUMMARY OF THE INVENTION

The present invention is based on the discovery of biomarkers useful for diagnosing and monitoring neuropsychiatric diseases. Neuropsychiatric diseases which may be diagnosed or monitored using the biomarkers of the invention include, without limitation: multiple sclerosis (especially focused on first attack multiple sclerosis), Neurologic Lyme Disease, and Chronic Fatigue Syndrome. Biomarkers identified for such neuropsychiatric diseases are listed herein in Tables 1m, 2m, 3, 4H-m, 4H-l, 4H-c, 5c, 6c, 7L, 8L, 9, and 10. As indicated in the tables presented herein, the presence or absence of at least one biomarker in a biological sample isolated from a patient is indicative of a positive diagnosis of a neuropsychiatric disease. As indicated herein, the identification of a plurality of biomarkers correlated with a particular disease may strengthen the confidence level of the diagnostic prediction. Biomarkers identified herein have also been assessed using software programs (such as, e.g., Ingenuity Pathway Analysis) to identify metabolic pathways implicated in disease etiology, maintenance, and/or progression. Multiple hits in a particular metabolic pathway underscore the potential importance of the pathway for the disease and direct therapeutic intervention toward appropriate modulation of same.

Accordingly, the present invention is directed to a method for diagnosing multiple sclerosis in a patient, the method comprising:
analyzing a biological sample isolated from the patient for expression of a panel of biomarkers including 14-3-3 epsilon protein, to determine if 14-3-3 epsilon protein is identified in the biological sample, wherein identification of 14-3-3 epsilon protein in the biological sample indicates a positive diagnosis of multiple sclerosis in the patient.

In another aspect, a method for diagnosing multiple sclerosis in a patient is presented, the method comprising: analyzing a biological sample isolated from the patient for expression of a panel of biomarkers, wherein the panel of biomarkers is set forth in any one of Tables 1m, 3m, 4H-m or 2m, to determine if at least two of the biomarkers listed in any one of Tables 1m, 3m, 4H-m or Table 2m, one of which being 14-3-3 epsilon protein, are identified in the biological sample, wherein identification of at least two of the biomarkers in the biological sample indicates a positive diagnosis of multiple sclerosis in the patient. In an embodiment thereof, the at least two biomarkers identified in the biological sample are present in the biological sample and set forth in Table 1m, 3m, or 4H-m, and the presence of the at least one biomarker indicates a positive diagnosis of multiple sclerosis. In another embodiment, the at least one biomarker identified in the biological sample is absent from the biological sample and set forth in Table 2m and the absence of the at least one biomarker indicates a positive diagnosis or is indicative of multiple sclerosis.

In accordance with the methods described herein, the biological sample may be cerebrospinal fluid, blood or a component thereof (e.g., plasma, serum, cells), tissue or tissue-related fluids, urine, or saliva.

Also encompassed herein is a method for diagnosing multiple sclerosis, the method comprising: analyzing a biological sample isolated from the patient to determine a first level of at least one of the biomarkers indicative of the presence of multiple sclerosis in the biological sample; and c) comparing the first level of the at least one of the biomarkers to a second level of the at least one of the biomarkers determined for a subject, wherein the subject does not have multiple sclerosis disease, and wherein at least a two-fold difference in the ratio of the first level to the second level indicates a positive diagnosis of multiple sclerosis. In a particular embodiment, the at least two-fold difference is an increase, which is indicative of a positive diagnosis of multiple sclerosis. In an alternative embodiment, the at least two-fold difference is a decrease, which is indicative of a positive diagnosis of multiple sclerosis. As described herein, in certain circumstances the subject is a healthy subject. In accordance with the invention, the subject may have a second neuropsychiatric disease, wherein the second neuropsychiatric disease differs from multiple sclerosis. Under such a circumstance, the two-fold difference in the ratio may be used to confer a differential diagnosis between multiple sclerosis and the second neuropsychiatric disease. This is a particularly useful feature of the present method when the clinical presentation multiple sclerosis and the second neuropsychiatric disease is similar.

Also encompassed herein are kits for diagnosing neuropsychiatric diseases. In a particular aspect, a kit for diagnosing multiple sclerosis is presented, wherein the kit comprises probes (e.g., antibodies or fragments or derivatives thereof) for detecting expression of at least one biomarker listed in Table 1m, 3m, 4H-m, or Table 2m, wherein detectable expression of at least one biomarker listed in Table 1m, 3m, or 4H-m is indicative of a positive diagnosis of multiple sclerosis and wherein an absence of detectable expression of at least one biomarker listed in Table 2m is indicative of a positive diagnosis of multiple sclerosis.

As described herein, the kits embodied herein may comprise probes for detecting expression of at least 2, 3, 4, 5, 6, 7, 8, 9, 10, 15, 20, 25, or 30 of the biomarkers listed in any one of Tables 1m, 3m, 4H-m or 2m, wherein the number of biomarkers listed in any one of Tables 1m, 3m, 4H-m or 2m establishes the upper limit of biomarkers detected by the kit. In other words, each of the Tables encompasses a maximum number of biomarkers as indicated therein and the number of biomarkers listed in the Tables may differ between each Table. In a particular embodiment, a kit may comprise probes for detecting expression of all of the biomarkers listed in any one of Tables 1m, 3m, 4H-m or 2m. In particular embodiments, the probes may be immobilized on a solid surface.

In another aspect, the invention is directed to a method for monitoring efficacy of a treatment for multiple sclerosis in a patient, wherein said method comprises isolating a first biological sample from the patient prior to initiating the treatment and isolating a second biological sample from the patient after initiating the treatment, analyzing the biological samples to determine the level of at least one of the biomarkers indicative of the presence of multiple sclerosis in the first and second biological samples, and comparing the level of the at least one of the biomarkers in the first and second biological samples, wherein a change in the level of the at least one of the biomarkers reflects the efficacy of the treatment. In one embodiment, wherein the presence of the at least one biomarker is associated with a positive diagnosis or presence of the disease, a detectable reduction in the level of the at least one biomarker following initiating the treatment indicates that the treatment is efficacious. In another embodiment, wherein the absence of the at least one biomarker is associated with a positive diagnosis or presence of the disease, a detectable increase in level in the at least one biomarker following initiating the treatment indicates that the treatment is efficacious.

In another aspect, the invention is directed to a method for monitoring disease progression of multiple sclerosis in a patient, wherein said method comprises isolating a first biological sample from the patient at an initial or first temporal window and isolating a second biological sample from the patient at a later (e.g., second) temporal window, analyzing the biological samples to determine the level of at least one of the biomarkers indicative of the presence of multiple sclerosis in the first and second biological samples, and comparing the level of the at least one of the biomarkers in the first and second biological samples, wherein a change in the level of the at least one of the biomarkers in the first and second biological samples reflects progression of the disease. In one embodiment, wherein the presence of the at least one biomarker is associated with a positive diagnosis or presence of the disease, a detectable increase in the level of the at least one biomarker at the later temporal time window relative to the initial temporal window indicates that the disease has progressed or worsened. In another embodiment, wherein the absence of the at least one biomarker is associated with a positive diagnosis or presence of the disease, a detectable decrease in the level of the at least one biomarker at the later temporal time window relative to the initial temporal window indicates that the disease has improved.

In one embodiment, the present invention relates to the identification and use of biomarkers in methods for diagnosing multiple sclerosis, particularly diagnosis of first attack multiple sclerosis. The invention also pertains to the use of these biomarkers in methods for assessing relapse of multiple sclerosis patients and disease progression in multiple sclerosis patients. Biomarkers identified in accordance with the invention are also useful for identifying treatments for and monitoring efficacy of treatment of patients with multiple sclerosis. As shown herein, the inventors have identified a plurality of biomarkers whose presence is indicative of multiple sclerosis; such biomarkers are listed in Tables 1m, 3m, or 4H-m. The inventors have also identified a plurality of biomarkers whose absence is indicative of multiple sclerosis; such biomarkers are listed in Table 2m.

Also encompassed herein are diagnostic signatures for multiple sclerosis. Such diagnostic signatures comprise biomarkers for diagnosing multiple sclerosis, wherein the presence or absence of at least one biomarker indicates a positive diagnosis of multiple sclerosis. In a particular embodiment, the diagnostic signature relates to the presence of at least one biomarker listed in Table 1m, 3m, or 4H-m and is indicative of a positive diagnosis of multiple sclerosis. In another embodiment, the diagnostic signature relates to the absence of at least one biomarker listed in Table 2m and is indicative of a positive diagnosis of multiple sclerosis.

### BRIEF DESCRIPTION OF THE DRAWINGS

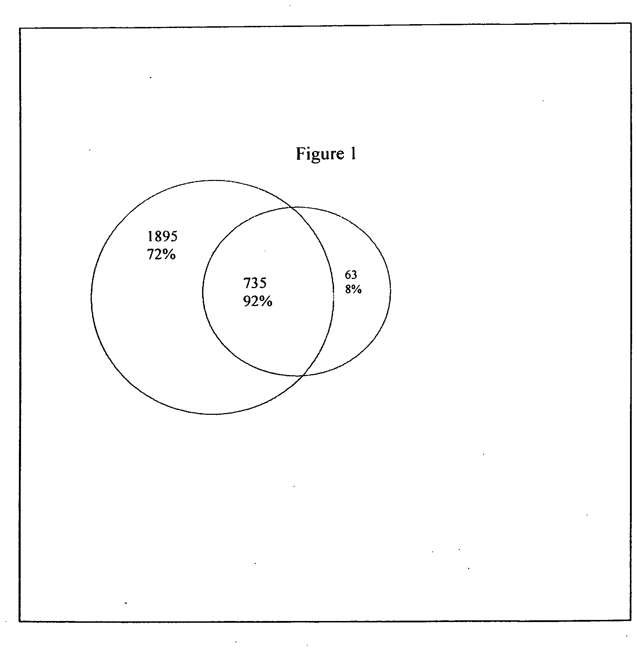

**Figure 1** shows a Venn diagram revealing the amount of overlap of the present dataset with a comparable dataset of proteins detected in the CSF of "normal clinical value" or "neurologic surrogate-normal" individuals who required a lumbar puncture for clinical reasons as reported by Zougman et al. [(2008) J Proteome Res 7: 386-399]. The large circle represents the 2630 proteins observed in the present comprehensive dataset of proteins detected in the CSF of normal individuals. The small circle represents the 798 proteins identified in the analysis by Zougman et al. [(2008) J Proteome Res 7: 386-399].

### DETAILED DESCRIPTION OF THE INVENTION

The present inventors had a unique opportunity to generate what may be the most comprehensive database of true normal CSF proteins to date. The inventors were able to do this because they had sufficient numbers and total volume of true normal CSF samples to employ immunoaffinity depletion, followed by extensive fractionation and high-resolution liquid chromatography (LC) separation and mass spectrometry (MS) analysis. The combination of the normal CSF samples, including a set of serial CSF samples, and advanced technology contribute to the uniqueness and value of the present study. Knowledge as to the normal CSF proteome, in turn, makes possible a meaningful comparison with disease proteomes (i.e., proteomes characteristic of a particular disease).

Until recently, technological limitations have prevented full characterization of the CSF proteome. Comprehensive analysis of CSF has been challenged by low protein levels (0.3 to 0.7 mg/ml) compared to plasma, protein concentration variability up to twelve orders of magnitude, potential masking of brain-specific proteins by highly abundant proteins [Rozek et al. (2007) J Proteome Res 6: 4189-4199], and limited access to an adequate number of appropriate biological samples. Despite some of these limitations, a number of earlier studies have provided increasing levels of characterization of the CSF proteome. For the most part, these studies have used pooled samples from patient populations with diseases or from people with normal CSF clinical laboratory values (chemistries, cell counts, and microbiology) who underwent lumbar puncture for investigation of neurological complaints. These CSF samples were used as substitutes or surrogates for true normals (healthy volunteers) possibly due to lack of availability of such normal CSF samples. Sickmann et al., [(2000) Electrophoresis 21: 2721-2728] used two-dimensional polyacrylamide gel electrophoresis (2D-PAGE) followed by mass spectrometry (MS) to identify close to 70 CSF proteins. Yuan et al., [(2005) Proteomics 5: 541-550] used matrix-assisted laser desorption ionization (MALDI) MS to identify 22 proteins in desalted CSF. Wenner et al., [(2004) J Proteome Res 3: 97-103] used 2D liquid chromatography (LC) coupled to tandem mass spectrometry (2D-LC-MS/MS) to identify 249 proteins in pooled CSF. Maccarrone et al. [(2004) Electrophoresis 25: 2402-2412], used immunodepletion techniques and shotgun LC-MS/MS, to identify more than 100 proteins in CSF from a patient with normal pressure hydrocephalus. More recently Zougman et al. [(2008) J Proteome Res 7: 386-399], using LC-MS/MS, reported 798 proteins in a pool of 6 patients with neurologic complaints that warranted a lumbar puncture, but whose subsequent clinical CSF laboratory values were reported as normal; for the purposes of this application these types of patients as referred to as neurologic surrogate-normals. A notable exception to use of surrogates was the work by Zhang et al. [(2005) Neurobiol Aging 26: 207-227], who used 2D-LC-MS/MS to identify 315 proteins in pools of CSF comparing healthy younger versus older individuals. Subsequently, with Xu et al. [(2006) Int Rev Neurobiol 73: 29-98], they analyzed CSF from the younger group with two different LC-MS/MS platforms and identified a combined total of 915 proteins. Pan et al., reported a total of 2594 CSF protein identifications from different combined (cumulative) results of several CSF studies with a focus on neurodegenerative diseases such as Alzheimer's disease [(2007) Proteomics 7: 469-473].

Towards the principal purpose of establishing a comprehensive list or proteome of normal CSF, the present inventors have prepared CSF samples from healthy normal people for analysis by using immunoaffinity depletion of abundant proteins (with masking potential) to enhance coverage and detection of low abundance proteins [Qian et al. (2009) J Proteome Res 8: 290-299]. It is noteworthy that the present inventors also analyzed the abundant sample fraction in parallel. Samples were then analyzed employing high throughput, high sensitivity, and high resolution nanocapillary liquid chromatography-mass spectrometry (LC-MS [Petyuk et al. (2007) Genome Res 17: 328-336] and LC-MS/MS [Qian et al. (2009) J Proteome Res 8: 290-299]). Pre-fractionation (immunoaffinity depletion chromatography) and ultra-high resolution nanocapillary LC separations were used to effectively reduce the sample complexity and concentration dynamic range (thereby reducing or eliminating the "masking" effect [Ramstrom et al. (2005) J Proteome Res 4: 410-416]), high efficiency ion transmission technologies (e.g., electrodynamic ion funnel [Shaffer et al. (1997) Rapid Commun Mass Spectrom 11: 1813-1817]) for highly sensitive global MS analysis, and the accurate mass and time (AMT) tag strategy [Smith et al. (2002) Proteomics 2: 513-523] for high-throughput analysis (e.g. of individual CSF samples) and accurate quantitation. The general approach for AMT tag generation and application as described herein has been successfully implemented for whole microbial [Lipton et al. (2002) Proc Natl Acad Sci U S A 99: 11049-11054] and mammalian tissue [Petyuk et al. (2007) Genome Res 17: 328-336] and plasma proteomes [Qian et al. (2009) J Proteome Res 8: 290-299; Qian et al. (2005) Mol Cell Proteomics 4: 700-709], but has not been previously applied to CSF from normal subjects.

As described herein, pooled CSF isolated from 11 normal healthy volunteers (8 women and 3 men, aged 24 to 55, median = 28 years) who reported their health as excellent or good and were taking no medications was examined. Standard clinical laboratory testing on their CSF was normal (none had more than 3 white blood cells/mm³ and protein levels ranged from 14 to 40 mg/dl with a median of 25 mg/dl). Also examined were pairs of individual serial CSF samples, obtained at least 4 weeks apart, from 10 additional normal healthy volunteers to assess the potential variability of particular CSF protein levels in an individual from one time point to another.

To illustrate the utility of such a normal database and how one clinical condition might be compared to another the present inventors also analyzed and compared one set of CSF samples to another set processed in the same manner to evaluate, among other issues, if there might be significant differences among different surrogate-normal groups.

In summary and as described in greater detail herein below, the present inventors applied immunoaffinity separation and high sensitivity and resolution liquid chromatography-mass spectrometry to examine CSF from healthy normal individuals. 2630 proteins in CSF from normal subjects were identified, of which 56% were CSF-specific (i.e., as determined using the techniques described herein), not found in the much larger set of 3654 proteins identified in plasma. The above-mentioned 2630 proteins identified in normal subjects were identified with high confidence, meaning that two or more peptides corresponding to a particular protein were required as confirmation that the particular protein was, indeed, present in normal CSF. The present inventors also examined CSF from groups like those of subjects previously examined by others as surrogates for normals, where neurologic symptoms warranted a lumbar puncture but where clinical laboratory were reported as normal. Statistically significant differences between their CSF proteins and those determined for non-neurological normals examined herein were noted. The inventors also examined CSF from 10 volunteer subjects who had lumbar punctures at least 4 weeks apart and found that there was little variability in CSF proteins in an individual as compared from subject to subject.

The present results represent the most comprehensive characterization of true normal CSF to date. This normal CSF proteome establishes a comparative standard and basis for investigations into a variety of diseases with neurological and psychiatric features. With this essential information, the present inventors were able to identify biomarkers diagnostic for a number of neuropsychiatric diseases. See Examples I-IV.

Accordingly, the present invention encompasses identification of biomarkers diagnostic for a number of neuropsychiatric diseases. As indicated in each of Tables 1-10, the present inventors have identified biomarkers, whose presence or absence as indicated herein is a positive indicator of the presence of a neuropsychiatric disease in a patient. Thus, the presence or absence of at least one biomarker for a neuropsychiatric disease serves as a diagnostic tool for clinicians. It is understood that a clinician will evaluate a patient as a whole based on known criteria well established in the field for diagnosis of a neuropsychiatric disease and utilize the diagnostic method of the present invention in conjunction with such known diagnostic criteria to establish a definitive diagnosis.

According to the present invention, the term "neuropsychiatric diseases" refers to, without limitation, multiple sclerosis and in particular first attack or early multiple sclerosis, Neurologic Lyme Disease, Chronic Fatigue Syndrome, and other diseases with similar features that may place them in the differential diagnosis in evaluating a patient.

According to the present invention, the term "multiple sclerosis" is used to describe the art-recognized disease characterized by inflammation, demyelination, oligodendrocyte death, membrane damage, other neurologic damage, and axonal death. Multiple sclerosis can be characterized as one of four main varieties as defined in an international survey of neurologists (Lublin and Reingold, 1996, Neurology 46(4):907-11), which are namely: Relapsing-Remitting multiple sclerosis (RRMS), Primary Progressive multiple sclerosis (PPMS), Secondary-Progressive multiple sclerosis (SPMS), Progressive-Relapsing multiple sclerosis (PRMS).

According to the present invention, the term "Neurologic Lyme Disease" is used to describe a range of neurologic or psychiatric manifestations that may be associated with Lyme disease. As referred to herein, "Neurologic Lyme Disease" encompasses the range from an acute neurologic syndrome in an acute case of active Lyme Disease and late manifestation of Lyme disease (both sometimes referred to as neuroborreliosis), to the more poorly defined post-treatment Lyme disease syndrome and "chronic Lyme Disease"

According to the present invention, the term "Chronic Fatigue Syndrome" is used to describe a constellation of symptoms including fatigue that approach those described in the 1994 proposed case definition [Fukuda et al, Annals of Internal Medicine, Vol. 121, December 15, 1994, pp. 953-959] that are subject to revision. In practice it is often a clinical diagnosis of exclusion.

As used herein, the terms "patient" or "subject" are used interchangeably. Such patients may have a neuropsychiatric disease or disorder, such as multiple sclerosis, CFS, and Neurologic Lyme Disease. With respect to multiple sclerosis, for example, the terms " multiple sclerosis patient", "a subject who has multiple sclerosis ", "a patient who has multiple sclerosis ", "a multiple sclerosis subject", and similar phrases, are intended to refer to subjects who have been diagnosed with multiple sclerosis.

The terms "healthy subject" or "normal subject", and similar phrases, are intended to refer to a subject who has not been diagnosed with a neuropsychiatric disease or disorder. A healthy subject has no other acute systemic disease or, at the least, has no detectable acute systemic disease. With respect to aspects of the invention directed to, for example, multiple sclerosis, the terms "healthy subject", "non- multiple sclerosis subject", "a subject who does not have multiple sclerosis ", "a patient who does not have multiple sclerosis ", and similar phrases, are intended to refer to a subject who has not been diagnosed with multiple sclerosis.

As used herein, the term "patient" or "subject" may refer to a mammal, including a human.

As used herein, the term "biological sample" includes a sample of any cell type or from any tissue or body fluid, body fluids including, but not limited to: cerebrospinal fluid (CSF), blood (whole blood, serum, plasma, or cellular components), saliva, urine, prostatic fluid, or fluid from any suitable tissue. In a particular embodiment, the biological sample is CSF. In another embodiment, the biological sample is blood or any component of blood (e.g., serum, plasma, cells, etc.).

The term "protein" is used herein to mean protein, polypeptide, oligopeptide or peptide. The terms "biologic marker", "biomarker" or "disease-associated protein" are used herein to refer to proteins associated with specific diseases or conditions, including proteins from organs or tissues ("organ-specific" or "tissue-specific" proteins) affected by a disease or condition. In accordance with the present invention, the presence or absence of a biomarker is positively correlated with, indicative of, or diagnostic for the presence of a disease or condition, such as a neuropsychiatric disease, in a patient. Also in accordance with the present invention, a relative increase in levels of a particular biomarker or biomarkers as compared to a control or normal subject can also be positively correlated with, indicative of, or diagnostic for the presence of a disease or condition, such as a neuropsychiatric disease, in a patient.

The present invention, furthermore, encompasses a plurality of biomarkers or a "biomarker signature" that is positively correlated with, indicative of, or diagnostic for the presence of a disease or condition, such as a neuropsychiatric disease, in a patient. Accordingly, the presence or absence of a biomarker signature is positively correlated with, indicative of, or diagnostic for the presence of a disease or condition, such as a neuropsychiatric disease, in a patient. A biomarker signature may also encompass a plurality of biomarkers that exhibit a relative increase or decrease in levels as compared to a healthy or normal subject (e.g., a control subject) and is positively correlated with, indicative of, or diagnostic for the presence of a disease or condition, such as a neuropsychiatric disease, in a patient.

A skilled practitioner would, moreover, appreciate that the presence or absence of a particular protein in a sample may be weakly indicative of disease, but not diagnostic, if noted as a single determinant. If, however, a plurality of such single determinants are noted in a biological sample, the combined detection of several weakly indicative determinants may serve to identify a strong combinatorial diagnostic indicator of disease. Furthermore, the single protein/determinant need not even approach the threshold of weak diagnostic by itself but in combination with the detection or absence of detection of another protein or proteins may serve as a strong combinatorial diagnostic indication of a disease state. Accordingly, also encompassed herein are combinatorial diagnostic indicators that are associated with a particular disease and not observed in healthy subjects or patients with other diseases.

Accordingly, selected sets of one, two, three, and more of the biomarkers of this invention (up to the number equivalent to all of the biomarkers, including any intervening number, in whole number increments, e.g., 1, 2, 3, 4, 5, 6 ...) can be indicative of a positive diagnosis or used as diagnostic indicators for methods and/or kits described herein. In one embodiment, larger numbers of the biomarkers identified herein are used in methods or kits of the invention, since the accuracy of the method or kit may improve as the number of biomarkers screened increases. With respect to aspects of the invention pertaining to evaluating therapeutic efficacy, the methods and kits of the present invention include evaluating whether administration of a therapeutic composition causes a change, either a transient change or a long term change, in one or more of the biomarkers; in two or more of the biomarkers; in three or more of the biomarkers; in four or more of the biomarkers; in five or more of the biomarkers, in six or more of the biomarkers, etc.

Also envisioned are sets of at least 1%, 2%, 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% of the biomarkers listed in any one of the tables presented herein, including Tables 1m, 2m, 3, 4H-m, 4H-l, 4H-c, 5c, 6c, 7L, 8L, and 9, wherein the envisioned sets can be indicative of a positive diagnosis or used as diagnostic indicators for methods and/or kits described herein. With respect to aspects of the invention pertaining to evaluating therapeutic efficacy, the methods and kits of the present invention include evaluating whether administration of a therapeutic composition causes a change, either a transient change or a long term change, in a percent of the biomarkers listed in any one of the Tables described herein.

Representative permutations of biomarkers and/or biomarker signatures are presented herein below for illustrative purposes. Based on the methods described herein, a biological sample isolated from a subject may be determined to exhibit any one of the following biomarker signatures, which are set forth in exemplary fashion, and are not to be viewed as limiting: a+; a-; a+, b+, c+; or a+, b-, c+; wherein "a" = specific protein "a", "b" = specific protein "b", "c" = specific protein "c", etc. and "+" indicates that the protein is present or quantitatively increased relative to subjects without disease, which could be clinically normal or could have a different disease (e.g., a different disease having overlapping clinical symptoms which is, therefore difficult to distinguish) and "-" indicates that the protein is absent or quantitatively decreased relative to subjects without disease, which could be clinically normal or could have a different disease (e.g., a different disease having overlapping clinical symptoms which is, therefore difficult to distinguish).

For example, the easiest identification of a protein biomarker is the presence of a protein associated with a disease or condition and not with other conditions that might be clinically confused with the disease under consideration. Variations to this scenario include the situation wherein a biomarker is present in an increased quantity or a decreased quantity compared to other conditions or controls. Although not a protein biomarker, an example of this is the presence of glucose in the blood in high quantities in diabetics compared to normal individuals who still have glucose present but not in elevated quantities. Another variation to the first scenario is where the functional biomarker is not just one protein but two or more in combination that can be present or absent or quantitatively different, wherein the ensemble defines its biomarker potential.

In some embodiments, a biomarker of the invention is a member of a biological pathway. As used herein, the term "precursor" or "successor" refers to molecules that precede or follow the biomarker in the biological pathway. Thus, once a biomarker is identified as a member of one or more biological pathways, the present invention can include additional members of the biological pathway that come before (are upstream of or a precursor of) or follow (are downstream of) the biomarker. Such identification of biological pathways and their members is within the skill of one in the art.

Also encompassed herein is the analysis of biomarkers identified and listed in the tables presented herein to identify metabolic pathways implicated in the pathogenesis, maintenance, and/or progression of a disease. Such analyses may utilize a variety of software programs, including but not limited to the commercially available Ingenuity Pathway Analysis. Multiple hits in a particular metabolic pathway underscore the potential importance of the pathway for the disease and direct therapeutic intervention toward appropriate modulation of same. Accordingly, the present methods encompass such analyses and the identification of metabolic pathways of potential significance in a particular disease. Knowing that, for example, activation of a metabolic pathway appears to be linked or associated with a particular disease presents the opportunity to test pharmaceutical modulators of the pathway (i.e., inhibitors) to determine if such modulators could be used as therapeutics for treatment of patients with the disease.

Metabolic pathways may, for example, be associated with several disease conditions based on a quantitative assessment. Examples of pathways that may be markedly involved in one condition and not another or others may also be found. As a specific illustrative example, a comparison of Chronic Fatigue Syndrome (CFS) to Neurologic Post Treatment Lyme disease syndrome (nPTLS), when individual patient samples were analyzed, revealed that four components (C1S, C4B, C1QB, C1QC) associated with activation of the complement cascade were differentially increased in abundance consistently across the nPTLS patients compared to CFS. In another example, a comparison between CFS and nPTLS patients, looking at the pooled results, revealed that the CDK5 signaling pathway was significantly enriched for proteins identified only in the pooled *CFS* proteome. This signaling pathway has been linked to Parkinson's (Smith et al. 2003. Proc. Natl. Acad. Sci. 100:13650) and Alzheimer's diseases (Lew et al. 1994. Nature 371:423). Another example is related to pathways that are involved in two conditions, that is, common to both but decreased in abundance in the two disease conditions of CFS and nPTLS, compared to normal, but at different levels. Quantitative distinguishing differences could still be found between the two conditions. A specific example relates to networks relevant to neurological function, such as axonal guidance, where the proteins in *CFS* were further decreased relative to *nPTLS.*

Also envisioned are panels of biomarkers comprising at least 1%, 2%, 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% of the biomarkers listed in any one of the tables presented herein, including Tables 1m, 2m, 3, 4H-m, 4H-1, 4H-c, 5c, 6c, 7L, 8L, and 9, wherein the envisioned panels can be used to assess a biological sample isolated from a patient to evaluate expression patterns of the biomarkers of the panel, which expression patterns are indicative of a positive diagnosis of a neuropsychiatric disease as described herein. Panels of biomarkers comprising at least one, two, three, and more of the biomarkers listed in any one of Tables 1m, 2m, 3, 4H-m, 4H-l, 4H-c, 5c, 6c, 7L, 8L, and 9 (up to the number equivalent to all of the biomarkers in a particular table, including any intervening number, in whole number increments, e.g., 1, 2, 3, 4, 5, 6 ...) are also envisioned herein. Any of the indicated panels may also be used to evaluate efficacy of a therapeutic regimen.

Polypeptide biomarkers may be isolated by any suitable method known in the art. Native polypeptide biomarkers can be purified from natural sources by standard methods known in the art such as chromatography, centrifugation, differential solubility or immunoassay. In one embodiment, polypeptide and metabolite markers may be isolated from a biological sample using standard techniques known in the art, for example, chromatographic methods or affinity purification using substrate-bound antibodies that specifically bind to the marker. As described herein, immunoaffinity depletion of abundant proteins (with masking potential) enhances coverage and detection of low abundance proteins. As indicated herein above, depletion yields separate fractions that are characterized independently.

In a positively-associated example, once a protein or peptide is identified (including one lacking name identification, such as a hypothetical protein), a less expensive platform or assay, as compared to mass spectrometry, can be devised or adapted. The ELISA assay is such an example where an antibody to the biomarker protein/peptide can be used to capture the target biomarker in the biological sample being detected. The sample may be CSF or blood, etc. Variations of this immuno-based technique may also be used such as, but not limited to, Western blots wherein a sample comprising a biomarker is transferred to a membrane and then subsequently allowed to interact with specific antibodies that are coupled to an enzyme (later to be exposed to a substrate that permits colorimetric visualization) or fluorescent material that permit visualization of one or more of the components or subcomponents (such as antigens) of the target biomarker. Alternatively, a noncoupled antibody can be used first and then a second antibody directed to this antibody, which has an enzyme or fluorescent tag attached thereto, can be used in a sandwich-like fashion. Another variation of this protein capturing technique could be multiplexing of several target biomarkers by using beads coated with multiple antibodies such as in the Luminex platform.

In another embodiment, once a disease protein/peptide biomarker is identified subsequent research may demonstrate that the body of disease subjects makes antibodies to the biomarker or biomarkers and the normal or other disease subject are negative for same. Under such circumstances, the presence of antibodies for a disease protein/peptide biomarker is indicative of disease. Experimentally, the presence of such disease specific antibodies is determined by using the disease protein/peptide biomarker as a binding target for the antibodies. This is a common indirect strategy for detection of infectious disease where the presence of an antibody indicates exposure to a foreign agent and in some cases the rise in titers (quantity of antibodies) over a short period of time indicates a very recent exposure.

As used herein, a polypeptide is referred to as "isolated" when it has been removed from its natural milieu (i.e., that has been subject to human manipulation), and can include purified polypeptides, partially purified polypeptides, synthetically produced polypeptides, and recombinantly produced polypeptides, for example. As such, "isolated" does not reflect the extent to which the polypeptide has been purified.

According to the present invention, the phrase "selectively binds to" refers to the ability of an antibody or antigen binding fragment thereof to preferentially bind to specified proteins. More specifically, the phrase "selectively binds" refers to the specific binding of one protein to another (e.g., an antibody or antigen binding fragment thereof to an antigen), wherein the level of binding, as measured by any standard assay (e.g., an immunoassay), is statistically significantly higher than the background control for the assay. For example, when performing an immunoassay, controls typically include a reaction well/tube that contain antibody or antigen binding fragment alone (i.e., in the absence of antigen), wherein an amount of reactivity (e.g., non-specific binding to the well) by the antibody or antigen binding fragment thereof in the absence of the antigen is considered to be background. Binding can be measured using a variety of methods standard in the art including enzyme immunoassays (e.g., ELISA), immunoblot assays, etc.).

As used herein, the term "specifically binding," refers to the interaction between binding pairs such as an antibody and an antigen with an affinity constant of at most 10⁻⁶ moles/liter, at most 10⁻⁷ moles/liter, or at most 10⁻⁸ moles/liter.

The present invention includes the use of any of the biomarkers as described herein (including genes or their RNA or protein products), as targets for the development or identification of therapeutic compositions and strategies for the treatment of neuropsychiatric diseases, such as for example, multiple sclerosis and/or relapsing multiple sclerosis.

Methods to measure biomarkers of this invention, include, but are not limited to: Western blot, immunoblot, enzyme-linked immunosorbant assay (ELISA), radioimmunoassay (RIA), immunoprecipitation, surface plasmon resonance, chemiluminescence, fluorescent polarization, phosphorescence, immunohistochemical analysis, liquid chromatography mass spectrometry (LC-MS), matrix-assisted laser desorption/ionization time-of-flight (MALDI-TOF) mass spectrometry, microcytometry, microarray, microscopy, fluorescence activated cell sorting (FACS), flow cytometry, laser scanning cytometry, hematology analyzer and assays based on a property of the protein including but not limited to DNA binding, ligand binding, or interaction with other protein partners. More particularly, high throughput, high sensitivity, and high resolution nanocapillary liquid chromatography-mass spectrometry (LC-MS), LC-MS/MS, pre-fractionation (immunoaffinity depletion chromatography) and ultra-high resolution nanocapillary LC separations, high efficiency ion transmission technologies (e.g., electrodynamic ion funnel), and the accurate mass and time (AMT) tag strategy for high-throughput analysis are described herein and known in the art.

As described herein, the term "probe" is used to refer to an agent that specifically binds to a biomarker listed in one of the tables presented herein. Suitable reagents for binding with a polypeptide corresponding to a biomarker of the invention include antibodies, antibody derivatives, labeled antibodies, antibody fragments, and the like. The term "probe" may also be used to refer to an agent that specifically binds to a nucleic acid sequence that encodes a biomarker listed in one of the tables presented herein. Suitable reagents for binding to a nucleic acid (*e.g.,* a genomic DNA, an mRNA, a spliced mRNA, a cDNA, or the like) include complementary nucleic acids.

In accordance with the present disclosure, the tables present information with which an ordinarily skilled practitioner can access the amino acid sequences of the proteins identified herein as biomarkers, as well as nucleic acid sequences encoding same. A stepwise protocol for identification of the amino acid sequences listed in the tables presented herein is as follows: access one of the publicly available databases such as http://www.ebi.ac.uk/IPI/IPIhelp.html or http://www.uniprot.org/ via the internet and enter the IPI number, or the protein name, or the Gene designation. For example, the first protein listed in Table 1m is designated "14-3-3 protein epsilon" and the corresponding Gene designation is YWHAE, and IPI designation of IPI00000816.1. Entering the IPI number, protein name, or Gene into one of the above publicly available databases will reveal the 255 amino acid sequence corresponding to "14-3-3 protein epsilon". The above protocol is a matter of routine practice in laboratories skilled in the art and can be performed for any of the proteins listed therein. Such information may be used to design probes for detection of any of the proteins listed therein or to identify commercially available probes for detection of any of the proteins listed therein. Primers for detection nucleic acid sequences encoding any of the proteins listed in the tables presented herein are also envisioned. Such primers may be used to detect expression or lack thereof of a biomarker or biomarker signature of the invention. The design of primers for detecting expression levels of RNA (e.g., mRNA) of a biomarker or biomarkers listed herein is a matter of routine practice with the nucleic acid sequence in hand as provided by publicly available websites such as those mentioned above. Such probes and primers are useful for the kits described herein.

The present invention also includes a method to diagnose a subject as having multiple sclerosis. In one embodiment, the method includes the steps of analyzing a biological sample isolated from the subject to determine if one or more of the biomarkers listed in Tables 1m, 3m, or 4H-m is present or if one or more of the biomarkers listed in Table 2m is absent, and diagnosing multiple sclerosis, wherein the presence (Tables 1m, 3m, or 4H-m) or absence (Table 2m) of one or more of these biomarkers indicates that the subject has multiple sclerosis. The present invention further encompasses circumstances wherein there is a change in the level/amount of a biomarker of the invention and such a change may also reflect the presence of multiple sclerosis or responsiveness to a therapeutic regimen.

The invention includes a kit for assessing the expression and/or expression levels of at least one of the biomarkers listed in the tables presented herein, whereby detecting the expression and/or expression levels of at least one of the biomarkers in a biological sample isolated from a patient is indicative of a positive diagnosis of the relevant disease to which the table in question pertains (e.g., MS, NLD, or CFS). The kit comprises a plurality of reagents, each of which is capable of binding specifically with a polypeptide (e.g., an antibody) or nucleic acid encoding same corresponding to a biomarker of the invention, *e.g.,* one of the proteins listed in any one of the tables presented herein. Suitable reagents for binding with a polypeptide corresponding to a biomarker of the invention include antibodies, antibody derivatives, labeled antibodies, antibody fragments, and the like. Suitable reagents for binding to a nucleic acid (*e.g.,* a genomic DNA, an mRNA, a spliced mRNA, a cDNA, or the like) include complementary nucleic acids. For example, the nucleic acid reagents may include oligonucleotides (labeled or non-labeled) fixed to a substrate, labeled oligonucleotides not bound with a substrate, pairs of PCR primers, molecular beacon probes, and the like.

The kit of the invention may optionally comprise additional components useful for performing the methods of the invention. By way of example, the kit may comprise fluids [*e.g.,* phosphate buffered saline (PBS) or SSC buffer] suitable for binding an antibody to a protein for which it is immunologically specific or for annealing complementary nucleic acids, one or more sample compartments, an instructional material which describes performance of a method of the invention, a positive control or controls, such as panel of proteins corresponding one of the panels set forth in any one of the tables presented herein or a biological sample isolated from a normal subject (a subject who does not manifest clinical symptoms of disease), or a biological sample isolated from a patient known to have the disease in questions, and the like.

The following protocols are provided to facilitate the practice of the present invention.

The following examples are put forth so as to provide those of ordinary skill in the art with a complete disclosure and description of how to make and use the assay, screening, and therapeutic methods of the invention, and are not intended to limit the scope of what the inventors regard as their invention. Efforts have been made to ensure accuracy with respect to numbers used (*e.g.,* amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### EXAMPLE I

### Materials and Methods

**Cerebrospinal Fluid (CSF) specimens.** All specimens had normal clinical laboratory values with respect to microbiology, chemistry (including protein levels), and cell counts (red blood cell were 0-10 / mm³ and white blood cells were 0-5/ mm³). Four sets of different types of normal CSF samples were analyzed. The first set, designated as true (healthy) normals was comprised of pooled CSF from 11 healthy normal individual volunteers from the United States (8 women and 3 men; aged 24 to 55 years with a median age of 28 years) was used for the comprehensive analysis using immunoaffinity depletion and 2D-LC-MS/MS. A second set, also true normals included pairs of serial CSF aliquots taken at least 4 weeks apart from 10 healthy volunteers from the United States (age 37-44 years; 5 males and 5 females). A third set, designated as non-neurologic surrogate-normals, was a pool of 200 subjects from Sweden (all without a neurologic or psychiatric disease, most who underwent lumbar puncture for non diagnostic reasons; over 90% were undergoing spinal anesthesia in preparation for orthopedic surgery (e.g. limbs-knees and hips)). Ages ranged from 16 to 65 years with a median of 44 years; 50:50 female:male. They were used in the direct LC-MS analysis using the AMT tag approach. These samples were collected on ice and cells removed by centrifugation [Bergquist et al. (2002) Mass Spectrom Rev 21: 2-15]. A fourth set, designated as neurologic surrogate-normals consisted of a pool of CSF from 10 people from Sweden with headaches (age 18-35 years; 8 female and 2 male) who had a lumbar puncture to investigate possible CNS infection, and who had normal CSF clinical laboratory values (hence designation surrogate-normal), was collected following the same protocol as the third set and analyzed in the same fashion as the second and third sets of normal CSF. CSF from this group was also subjected to centrifugation to remove cells. CSF from this group was collected following the same protocol as the pool of the 200 non-neurological surrogate-normals. The protein concentrations were determined by Coomassie Plus protein assay (Pierce, Rockford, IL) using a bovine serum albumin standard.

**Immunoaffinity depletion of 14 high-abundance CSF proteins.** A total of 18 mL of the pooled CSF sample (from the 11 healthy volunteers) was subjected to the separations of 14 high-abundance proteins (albumin, IgG, α₁-antitrypsin, IgA, IgM, transferrin, haptoglobin, α₁-acid glycoprotein, α₂-macroglobulin, apolipoprotein A-I, apolipoprotein A-II, fibrinogen, C3 and apolipoprotein B) using a 12.7 × 79.0 mm Seppro® IgY14 LC10 affinity LC column (Sigma, St Louis, MO) on an Agilent 1100 series HPLC system (Agilent, Palo Alto, CA), followed the protocols described previously [Liu et al. (2006) Mol Cell Proteomics 5: 2167-2174].

**Protein digestion.** The CSF proteins were incubated in 8 M urea and 10 mM dithiothreitol at 37°C for 60 min, followed by alkylation with 40 mM iodoacetamide in the dark for 30 min at room temperature. The samples were diluted 10-fold with 50 mM ammonium bicarbonate (pH 8) and 1 mM CaCl₂, and digested for 3 h at 37 °C using sequencing grade, modified porcine trypsin (Promega, Madison, WI) at a trypsin/protein ratio of 1:50. Sample cleanup was achieved using a 1-mL SPE C18 column (Supelco, Bellefonte, PA) as described previously [Liu et al. (2006) Mol Cell Proteomics 5: 1899-1913]. The final peptide concentration was determined by BCA assay (Pierce). All tryptic digests were snap frozen in liquid nitrogen and stored at -80 °C.

**Strong cation exchange (SCX) fractionation.** For tryptic digests of the IgY14 bound and flow-through fractions (the first set normal CSF pool from BN), 300 µg of tryptic peptides from CSF samples were resuspended in 300 µL 10 mM ammonium formate, 25% acetonitrile and fractionated by strong cation exchange chromatography as described previously [Liu et al. (2006) Mol Cell Proteomics 5: 1899-1913]. A total of 30 fractions were collected for each sample with each fraction being lyophilized prior to reversed-phase LC-MS/MS analysis.

**Reversed-phase capillary LC-MS/MS and LC-MS analysis.** The SCX fractions were analyzed using a custom-built automated four-column high pressure nanocapillary LC system coupled on-line to either a linear ion trap mass spectrometer (LTQ; ThermoFisher) or a linear quadrupole ion trap-orbitrap mass spectrometer (LTQ-Orbitrap, ThermoFisher), both modified in-house with an electrodynamic ion funnel [Shaffer et al. (1997) Rapid Commun Mass Spectrom 11: 1813-1817], via an electrospray ionization interface manufactured in-house. The reversed-phase separation was performed as described previously [Liu et al. (2006) Mol Cell Proteomics 5: 1899-1913]. To analyze the SCX fractions of the IgY14 bound fraction, the LTQ mass spectrometer was operated in a data-dependent MS/MS mode (m/z 400-2000) in which a full MS scan was followed by 10 MS/MS scans. The ten most intensive precursor ions were dynamically selected in the order of highest intensity to lowest intensity and subjected to collision-induced dissociation using a normalized collision energy setting of 35% and a dynamic exclusion duration of 1 min. The heated capillary was maintained at 200 °C, while the ESI voltage was kept at 2.2 kV. The SCX fractions of the IgY14 flow-through fraction, which are enriched with lower abundance proteins, were analyzed by the LTQ-Orbitrap instrument operated in a data-dependent MS/MS mode with survey full scan MS spectra (m/z 400-2000) acquired in the orbitrap with resolution of 30,000 at m/z 400 (ion accumulation target: 1,000,000), followed by MS/MS of the 10 most intense ions. In the case of label-free quantitation using the unfractionated CSF samples (the second and third set of normal CSF and the headache CSF), the LTQ-Orbitrap MS was operated in the data dependent mode with survey full scan spectra (m/z 400-2000) acquired in the orbitrap with resolution of 60,000 at m/z 400 (accumulation target: 1,000,000). The six most intense ions were sequentially isolated for fragmentation and detection in the linear ion trap.

**Data analysis.** The LTQ LC-MS/MS raw data were converted into .dta file by Extract_MSn (version 3.0) in Bioworks Cluster 3.2 (Thermo) and the SEQUEST algorithm (version 27 revision 12) was used to independently search all the MS/MS spectra against the human International Protein Index (IPI) database with a total of 69,731 total protein entries (Version 3.40, released at February 7, 2008). The search parameters used were: 3-Da tolerance for precursor ion masses and 1-Da tolerance for fragment ion masses with no enzyme restraint and a maximum of 2 missed tryptic cleavages. Static carboxyamidomethylation of cysteine and dynamic oxidation of methionine were used during the database search. LTQ-Orbitrap MS/MS data were first processed by an in-house software DeconMSn [Mayampurath et al. (2008) Bioinformatics 24: 1021-1023] to accurately determine the monoisotopic mass and charge state of parent ions, followed by SEQUEST search against the IPI database in the same fashion, except that 0.1-Da tolerance for precursor ion masses and 1-Da tolerance for fragment ion masses were used. A set of criteria considering the cross correlation score (Xcorr) and delta correlation (ΔCn) values along with tryptic cleavage and charge states were developed using the decoy database approach and applied for filtering the raw data to limit false positive identifications to <1% at the peptide level [Peng et al. (2003) J Proteome Res 2: 43-50; Elias et al. (2007) Nat Methods 4: 207-214; Qian et al. (2005) J Proteome Res 4: 53-62]. For the LTQ-Orbitrap data, the distribution of mass deviation (from the theoretical masses) was first determined as having a standard deviation (σ) of 2.05 part per million (ppm), and peptide identifications with mass error of greater than 3σ were filtered out. In general, slightly lower Xcorr cutoff values were used when combined with ΔCn and the mass error constraint to achieve the same level of false positive rate (<1%). For peptides identified by both LTQ-Orbitrap (IgY14 flow-through fraction) and LTQ (IgY14 bound fraction) analyses, the database matching scores are shown only for the LTQ-Orbitrap analysis, along with their mass errors.

The AMT tag strategy [Smith et al. (2002) Proteomics 2: 513-523] was used for identifying and quantifying LC-MS features measured by LTQ-Orbitrap. The filtered MS/MS peptide identifications obtained from the LTQ and LTQ-Orbitrap analyses of CSF samples were included in an AMT tag database with their theoretical mass and normalized elution time (NET; from 0 to 1) recorded. LC-MS datasets were then analyzed by in-house software VIPER [Monroe et al. (2007) Bioinformatics 23: 2021-2023] that detects features in mass-NET space and assigned them to peptides in the AMT tag database [Zimmer et al. (2006) Mass Spectrom Rev 25: 450-482]. A 11-Da shift strategy analogous to the decoy database approach used for LC-MS/MS identification of peptides was applied for estimating the false discovery rate of the AMT analysis as previously described [Petyuk et al. (2007) Genome Res 17: 328-336]. A false positive rate of <4% was estimated for each of the LC-MS data sets. The resulting lists of peptides from 2D-LC-MS/MS or direct LC-MS analysis was further analyzed by ProteinProphet software [Nesvizhskii et al. (2003) Anal Chem 75: 4646-4658] to remove redundancy in protein identification as described previously [Liu et al. (2006) Mol Cell Proteomics 5: 1899-1913].

Data normalization and quantification of the changes in protein abundance between the normal and headache CSF samples were performed and visualized using in-house software DAnTE [Polpitiya et al. (2008) Bioinformatics 24: 1556-1558]. Briefly, peptide intensities from the LC-MS analyses were log2 transformed and normalized using a mean central tendency procedure. Peptide abundances were then "rolled up" to the protein level employing the R-rollup method (based on trends observed at peptide level) implemented in DAnTE. ANOVA and clustering analyses were also performed using DAnTE. Gene ontology annotation was performed using a software tool STRAP [Bhatia et al. (2009) Anal Chem 81: 9819-9823]. The final distribution charts were generated using Excel.

### Results

A comprehensive analysis of the CSF proteome from healthy normal individuals is described herein and presents the foundation for future investigation on this biological fluid which may be highly reflective of the status of the brain and central nervous system. The results described herein make available a comprehensive coverage of CSF proteins from normal healthy individuals. From the pool of 11 CSF samples from healthy volunteers, the present inventors identified with high confidence a total of 19,051 tryptic peptides, covering 2630 non-redundant proteins, with 1506 having at least two peptide identifications. The immunoaffinity-based partitioning generated a separate bound fraction consisting of the 14 most abundant proteins and their potential associated proteins, and a flow-through fraction enriched with the less abundant proteins in CSF. Similar to plasma, the bound fraction represents approximately 95% of the total protein mass. Both fractions were subjected to 2D-LC-MS/MS analysis.

The set of 2630 CSF proteins, and a comprehensive set of 3654 proteins from our previous plasma database [Liu et al. (2006) Mol Cell Proteomics 5: 1899-1913], showed very similar distribution of gene ontology terms in biological process and molecular function, but different distributions with respect to cellular component: a total of approximately 35% of the CSF proteins are from plasma membrane, cell surface or extracellular space, while blood plasma has a total of 28% of proteins in those three categories; there are also fewer CSF proteins derived from the nucleus and cytoplasm (10% versus 15% and 11% versus 16%, respectively. Importantly, nearly 56% of the proteins are CSF-specific and are not present in our larger plasma database of 3654 proteins, also analyzed by LC-MS/MS. This is notable because the acquisition and analysis conditions likely favored the set of plasma proteins as opposed to the CSF proteins. This is because more proteins were likely available for detection in the plasma of burn patients due to severe tissue leakage, and there was the additional dimension of sample fractionation via enrichment of cysteinyl and N-linked glycopeptides. It is noteworthy that this cannot be viewed as a head-to-head comparison in light of the differences in sample type and conditions, extensiveness of fractionation and MS instrumentation.

Comparison between proteins detected in this study and those (which we have termed neurologic surrogate-normals) from the CSF study by Zougman et al., reveals a 92% overlap (see Figure 1.

In order to assess the CSF protein variability from serial sample collections, the present inventors examined individual (non-pooled) samples from another group of 10 healthy volunteers (5 male, 5 female; age range 37-44 years old) who had two CSF samples obtained at least 4 weeks apart using the AMT tag approach. Inter-subject differences were far greater than intra-subject differences. Statistical tests of variance of differences (ANOVA) were performed for these data sets based on different factors (e.g., subject, gender, and time of sampling), followed by unsupervised hierarchical clustering analysis of the statistically significant proteins (p-value <0.01). It is clear that human heterogeneity is the major factor responsible for inter-sample differences; clustering of the "significant" proteins could not distinguish corresponding groups based on the other factors defined in the ANOVA analysis (i.e., gender and time of sampling), except for "subject" (i.e., normal volunteers).

As an example of how CSF proteomic databases may be used to better understand disease states, the present inventors compared the proteomes of two similarly processed (see Methods) pooled samples of patients using the mass spectrometry strategy [Smith et al. (2002) Proteomics 2: 513-523]. The first set, considered as neurologic surrogate-normals was a pool of 10 headache patients. CSF had been obtained to evaluate the possibility of a CNS infection or bleed but all clinical results were normal. The second set, considered as non-neurologic surrogate-normals, was a pool of 200 subjects (without a neurologic disease, who underwent lumbar puncture for non diagnostic reasons; over 90% were undergoing spinal anesthesia in preparation for orthopedic surgery (limbs-knees and hips)). Significantly distinct results were found between each group. Specifically, 191 ± 7 and 211 ± 8 non-redundant proteins were identified from the 3 replicates of each data set. Statistical analysis comparing these CSF data sets showed that the neurologic surrogate-normal CSF pool had distinctive quantitative differences compared to the non-neurologic surrogate normal pool (22 proteins with p-value <0.01 by ANOVA). Unsupervised hierarchical clustering of abundances of all proteins clearly separates these two groups. One interesting difference was the identification and quantification of certain hemoglobin isoforms, which were among significantly changed proteins identified by the statistical analysis, in the neurologic surrogate-normal (headache) samples. Although Zougman et al. [(2008) J Proteome Res 7: 386-399] previously identified these same proteins in a qualitative analysis of their neurologic surrogate-normal samples, the present study reveals that these isoforms were increased by about ten-fold on average. The differences found in the present study suggest that it would be attractive to extend these studies with immunoaffinity depletion applied to different defined categories of headache subjects.

### Summary

The present study provides the most comprehensive CSF protein coverage and list reported to date for healthy normal individuals including serial lumbar punctures. [Schutzer et al. (2010) Establishing the Proteome of Normal Human Cerebrospinal Fluid, PLoS One 5(6): e10980]. The protein set has immediate utility for investigators interested in using CSF to study neurological or psychiatric diseases. As evidenced in Examples II-IV, it serves as a normative base to which disease states may be compared. The present study also suggests that CSF protein variability over a short time may be relatively limited in an individual and thus functionally useful.

The other major previous investigations of CSF from healthy individuals were published by Zhang et al. [(2005) Neurobiol Aging 26: 207-227] and updated by part of that group, Xu et al. [(2006) Int Rev Neurobiol 73: 29-98]. What began as detection of approximately 315 proteins was expanded to 915 using different mass spectrometry methods. Interestingly Xu et al. [(2006) Int Rev Neurobiol 73: 29-98] stated that they believed their coverage of the normal CSF was insufficient because they were unable to detect two well known CSF proteins, α-synuclein [Yuan et al. (2005) Proteomics 5: 541-550] and gelsolin [Yuan et al. (2002) Electrophoresis 23: 1185-1196]. The present methods and approach differed from these references, and included a rigorous separation of abundant from less abundant proteins to mitigate the masking effect of the most abundant proteins, as well as high-resolution LC coupled to MS/MS analysis for highly efficient peptide identification. The present inventors did identified 2630 proteins in total, including α-synuclein and gelsolin.

Due to the challenge in obtaining CSF from healthy people, most previous studies may have used CSF from "surrogate-normals," that is CSF collected from people with neurological complaints such as headache but with normal clinical CSF laboratory values. As described herein, the present inventors compared proteomes of two different surrogate-normal groups. Significant differences were noted between the two groups. Accordingly, this study supports the potential usefulness of the "true" normal human CSF proteome data library as an invaluable tool in investigating pathophysiological abnormalities in neurological and psychiatric disorders.

Proteomic databases can be used in several ways. The present inventors' perspective with regard to using these proteomic databases for studying diseases involves a stepwise strategy. The first step is a comparison of pooled samples representative of the disease to normal subjects or a comparator disease. The second step involves the selection of specific candidate proteins. The selection of candidate proteins is not likely to be predicted in advance and may require bioinformatic strategies and knowledge related to the disease under study. A third step would involve analysis of the individual samples contributing to the pool to ascertain how many of the samples actually contained one or more of the candidate proteins. This step provides a check in the event that a single individual in the pool disproportionately contributes a protein compared to other subjects. The results would be subjected to statistical analyses in accordance with standard practice. In the case of a search for biomarker proteins, an objective is to select those that meet clinically useful criteria, such as presence, absence or relative abundance in a large percentage of disease subjects and not so in most subjects without the disease under consideration. The fourth step would involve verification of the previous results using independent individual samples with the same disease. A final validation step may involve analyzing a larger number of subjects with the disease and controls using assays targeted to the candidate proteins. In contrast to the discovery phases, it would be advantageous, if feasible, to use assay platforms already having wide clinical use. Immunobased assays such as ELISA and Western blots may serve this purpose being relatively inexpensive. Steps 3 and 4 will likely employ a type of mass spectrometry which targets selected candidate proteins, such as Multiple Reaction Monitoring (MRM) using triple quadrupole instrumentation.

The availability of the data presented here, detailing the normal human CSF proteome, should prove to be a critical base on which to compare proteins, both qualitatively and quantitatively, in studies of patients with a variety of neurological or psychiatric diseases.

### EXAMPLE II

### Multiple Sclerosis

In the experimental analysis of data on documented first attack Multiple Sclerosis subjects, long lists of proteins found in these subjects (data not shown) and small subset lists as well as a list of those proteins that appear to be absent (below the level of detection) have been found. Specifically:
1) Table 1m shows Cerebrospinal Fluid (CSF) Proteins present only in Multiple Sclerosis patients compared to Surrogate Non-Neurologic Normals and to Surrogate Neurologic Normals
   (Headaches). This is a Qualitative List of CSF Proteins Present in the Pooled First Attack Multiple Sclerosis Patients compared to the combined lists of Proteins Found in a Similarly Analyzed Group of Non-neurologic Surrogate Normal Subjects and Proteins Found in Neurologic Surrogate-Normals (Headache subjects with normal CSF clinical laboratory values).
2) Table 2m shows a Qualitative List of CSF Proteins Absent only in the Pooled First Attack Multiple Sclerosis Patients compared to the combined lists of a) Proteins Found in a similarly Analyzed Group of Non-neurologic Surrogate Normal Subjects (both groups analyzed without immunoaffinity depletion of most abundant proteins) and b) Proteins Found in an expanded list of True Normals (where immunoaffinity depletion was applied).
3) Table 3m shows a subset of CSF Proteins Present In Multiple Sclerosis not in combined lists of proteins from Normals or Headaches (immunoaffinity depletion was not applied)
4) Table 4H-m shows identified CSF proteins in first attack Multiple Sclerosis that have high potential to be candidate biomarkers because their function is not known and they remain unknown (referred to as a hypothetical or uncharacterized protein).

**Table 1m. Cerebrospinal Fluid (CSF) Proteins present only in Multiple Sclerosis patients compared to Surrogate Non-Neurologic Normals AND Surrogate Neurologic Normals (Headaches): Qualitative List of CSF Proteins Present in the Pooled First Attack Multiple Sclerosis Patients compared to the combined lists of Proteins Found in a Similarly Analyzed Group of Non-neurologic Surrogate Normal Subjects and Proteins Found in Neurologic Surrogate-Normals (Headaches subjects with normal CSF clinical laboratory values). IAD = immunoaffinity depletion**

| **MinOfORF** | **CSF Proteins Present In MS not Normals or Headaches (no IAD)** | **Gene** |
|---|---|---|
| IPI:IPI00000816.1 | 14-3-3 protein epsilon | YWHAE |
| IPI:IPI00001516.1 | Isoform Long of Protocadherin alpha C2 precursor | PCDHAC2 |
| IPI:IPI00001610.1 | Insulin-like growth factor IA precursor | IGF1 |
| IPI:IPI00001895.1 | Isoform 1 of Protocadherin-8 precursor | PCDH8 |
| IPI:IPI00006444.1 | Isoform 1 of Sodium/potassium/calcium exchanger 2 precursor | SLC24A2 |
| IPI:IP100006543.2 | Complement factor H-related 5 | CFHR5 |
| IPI:IPI00007778.1 | Di-N-acetylchitobiase precursor | CTBS |
| IPI:IPI00008994.2 | Isoform 1 of Protein NDRG2 | NDRG2 |
| IPI:IPI00012102.1 | N-acetylglucosamine-6-sulfatase precursor | GNS |
| IPI:IPI00012283.2 | Isoform 1 of Semaphorin-3B precursor | SEMA3B |
| IPI:IPI00012792.1 | Cadherin-5 precursor | CDH5 |
| IPI:IPI00015479.5 | UPF0454 protein C12orf49 precursor | C12orf49 |
| IPI:IPI00015911.1 | Dihydrolipoyl dehydrogenase, mitochondrial precursor | DLD |
| IPI:IPI00016666.1 | Metallothionein-3 | MT3 |
| IPI:IPI00017569.5 | Fas apoptotic inhibitory molecule 2 | FAIM2 |
| IPI:IPI00017704.3 | Coactosin-like protein | COTL1 |
| IPI:IPI00021833.1 | Isoform Long of Platelet-derived growth factor A chain precursor | PDGFA |
| IPI:IPI00025365.1 | Isoform Long of Endothelin-3 precursor | EDN3 |
| IPI: IPI00026569.3 | HLA class I histocompatibility antigen, A-1 alpha chain precursor | HLA-A |
| IPI:IPI00026946.2 | Neuronal pentraxin-2 precursor | NPTX2 |
| IPI:IPI00027765.1 | retbindin isoform 2 | RTBDN |
| IPI:IPI00029131.1 | Neuroendocrine convertase 2 precursor | PCSK2 |
| IPI:IPI00030877.2 | 15 kDa selenoprotein isoform 1 precursor | SEP15 |
| IPI:IPI00060310.4 | Phospholipase D4 | PLD4 |
| IPI:IPI00152540.3 | Isoform 1 of CD109 antigen precursor | CD109 |
| IPI:IPI00157414.3 | Ectonucleotide pyrophosphatase/phosphodiesterase family member 6 precursor | ENPP6 |
| IPI:IPI00163187.10 | Fascin | FSCN1 |
| IPI:IPI00171411.4 | Golgi phosphoprotein 2 | GOLM1 |
| IPI:IPI00172636.5 | Isoform Delta 6 of Calcium/calmodulin-dependent protein kinase type II delta chain | CAMK2D |
| IPI:IPI00180707.8 | Isoform 1 of FRAS1-related extracellular matrix protein 2 precursor | FREM2 |
| IPI:IPI00217542.4 | Putative uncharacterized protein LOCI30576 | LOC 130576 |
| IPI:IPI00217966.7 | Isoform 1 of L-lactate dehydrogenase A chain | LDHA |
| IPI:IPI00292550.2 | Isoform 1 of Polypeptide N-acetylgalactosaminyltransferase 13 | GALNT13 |
| IPI:IPI00294705.1 | Papilin | PAPLN |
| IPI:IPI00298547.3 | Protein DJ-1 | PARK7 |
| IPI:IPI00298793.4 | Beta-mannosidase precursor | MANBA |
| IPI:IPI00329688.2 | Protein YIPF3 | YIPF3 |
| IPI:IPI00334666.2 | Isoform 1 of Receptor-type tyrosine-protein phosphatase N2 precursor | PTPRN2 |
| IPI:IPI00337548.5 | Cell growth regulator with EF hand domain protein 1 | CGREF1 |
| IPI:IPI00376394.4 | Sulfhydryl oxidase 2 precursor | QSOX2 |
| IPI:IPI00382426.1 | Ig lambda chain V-II region TRO | |
| IPI:IPI00382447.1 | Ig lambda chain V-VI region AR | |
| IPI:IPI00382476.1 | Ig heavy chain V-III region WEA | |
| IPI:IPI00382482.1 | Ig heavy chain V-III region CAM | |
| IPI:IPI00382490.1 | Ig heavy chain V-III region BUR | |
| IPI:IPI00384402.1 | Myosin-reactive immunoglobulin kappa chain variable region (Fragment) | |
| IPI:IPI00385143.1 | Microfibrillar protein 2 (Fragment) | |
| IPI:IPI00386131.1 | Ig kappa chain V-III region IARC/BL41 precursor | |
| IPI:IPI00387096.1 | Ig kappa chain V-I region Kue | |
| IPI:IPI00387101.1 | Ig kappa chain V-I region Scw | |
| IPI:IPI00387113.1 | Ig kappa chain V-III region B6 | |
| IPI:IPI00419442.3 | IGLV6-57 protein | IGLV6-57 |
| IPI:IPI00455667.5 | hypothetical protein LOC402665 | LOC402665 |
| IPI:IPI00465255.6 | Isoform 1 of Proline-rich acidic protein 1 precursor | PRAP1 |
| IPI:IPI00748265.2 | Rheumatoid factor RF-ET13 | |
| IPI:IPI00816737.1 | Rheumatoid factor D5 heavy chain (Fragment) | |
| IPI:IPI00829810.1 | Uncharacterized protein ENSP00000375027 | |
| IPI:IPI00854624.1 | Uncharacterized protein ENSP00000375043 | |
| IPI:IPI00854745.1 | Uncharacterized protein ENSP00000375019 | |

**Table 2m. Qualitative List of CSF Proteins Absent only in the Pooled First Attack Multiple Sclerosis Patients compared to the combined lists of a) Proteins Found in a similarly Analyzed Group of Non-neurologic Surrogate Normal Subjects (both groups analyzed without immunoaffinity depletion (IAD) of most abundant proteins) and b) Proteins Found in an expanded list of True Normals (where immunoaffinity depletion was applied).**

| MinOfORF=Non redundant IPI designation | | |
|---|---|---|
| **MinOfORF** | **Gene** | **CSF Proteins Not Present in MS but Present in Combined Normals and Headaches (no IAD)** |
| IPI:IPI00000024.1 | PCDH1 | Isoform 1 of Protocadherin-1 precursor |
| IPI:IPI00001477.1 | DDR1 | Isoform 1 of Epithelial discoidin domain-containing receptor 1 precursor |
| IPI:IPI00003176.1 | HTRA1 | Serine protease HTRA 1 precursor |
| IPI:IPI00003648.2 | PVRL1 | Isoform Delta of Poliovirus receptor-related protein 1 precursor |
| IPI:IPI00004946.8 | CXCL16 | chemokine (C-X-C motif) ligand 16 |
| IPI:IPI000 10471.5 | LCP1 | Plastin-2 |
| IPI:IPI00021263.3 | YWHAZ | 14-3-3 protein zeta/delta |
| IPI:IPI00021856.3 | APOC2 | Apolipoprotein C-II precursor |
| IPI:IPI00022333.1 | BAI1 | Brain-specific angiogenesis inhibitor 1 precursor |
| IPI:IPI00024570.1 | SEMA3G | Semaphorin-3G precursor |
| IPI:IPI00025155.1 | FSTL3 | Follistatin-related protein 3 precursor |
| IPI:IPI00029193.1 | HGFAC | Hepatocyte growth factor activator precursor |
| IPI:IPI00029343.2 | CNTNAP2 | Isoform 1 of Contactin-associated protein-like 2 precursor |
| IPI:IPI00169383.3 | PGK1 | Phosphoglycerate kinase 1 |
| IPI:IPI00216171.3 | ENO2 | Gamma-enolase |
| IPI:IPI00218570.6 | PGAM2 | Phosphoglycerate mutase 2 |
| IPI:IPI00218834.9 | FCGR3A | Low affinity immunoglobulin gamma Fc region receptor III-A precursor |
| IPI:IPI00219425.3 | PVR | Isoform Beta of Poliovirus receptor precursor |
| IPI:IPI00297040.2 | SPINK6 | Serine protease inhibitor Kazal-type 6 precursor |
| IPI:IPI00306710.2 | CHRD | Isoform 1 of Chordin precursor |
| IPI:IPI00328703.1 | OAF | Out at first protein homolog precursor |
| IPI:IPI00329775.7 | CPB2 | Isoform 1 of Carboxypeptidase B2 precursor |
| IPI:IPI00385980.8 | ROBO2 | ROBO2 isoform a |
| IPI:IPI00387119.1 | | Ig kappa chain V-III region POM |
| IPI:IPI00411680.8 | PCMT1 | Isoform 1 of Protein-L-isoaspartate(D-aspartate) O-methyltransferase |
| IPI:IPI00444605.1 | | CDNA FLJ45296 fis, clone BRHIP3003340, moderately similar to Actin, alpha skeletal muscle 2 |
| IPI:IPI00456736.4 | RGMB | Isoform 1 of RGM domain family member B precursor |
| IPI:IPI00479116.1 | CPN2 | Carboxypeptidase N subunit 2 precursor |
| IPI:IPI00554474.3 | LOC284297 | Hypothetical LOC284297 |

**Table 3m. A subset table of Table 1 illustrating those CSF proteins identified that have high potential as candidate biomarkers.**

| MinOfORF = Non redundant IPI designation | | |
|---|---|---|
| IAD = Immunoaffinity depletion | | |
| **MinOfORF** | **CSF Proteins Present In Multiple Sclerosis not in combined lists of proteins from Normals or Headaches (no IAD)** | **GENE** |
| IPI:IPI00001516.1 | Isoform Long of Protocadherin alpha C2 precursor | PCDHAC2 |
| IPI:IPI00026569.3 | HLA class I histocompatibility antigen, A-1 alpha chain precursor | HLA-A |
| IPI:IPI00382426.1 | Ig lambda chain V-II region TRO | |
| IPI:IPI00382447.1 | Ig lambda chain V-VI region AR | |
| IPI:IPI00382490.1 | Ig heavy chain V-III region BUR | |
| IPI:IPI00829810.1 | Uncharacterized protein ENSP00000375027 | |
| IPI:IPI00854624.1 | Uncharacterized protein ENSP00000375043 | |
| IPI:IPI00854745.1 | Uncharacterized protein ENSP00000375019 | |

**Table 4H.** This Table is divided into 3. The tables show identified CSF proteins that have high potential to be candidate biomarkers because their function is not known and they remain unknown (referred to as a hypothetical or uncharacterized protein).

**Table 4H-m is a subset of Table 1 and Table 3 containing those hypothetical/uncharacterized CSF proteins identified in Multiple Sclerosis**

| IAD= Immunoaffinity depletion | | |
|---|---|---|
| MinOfORF=Non redundant IPI designation | | |
| **MinOfORF** | **CSF Proteins Present In Multiple Sclerosis but not Normals or Headaches (no IAD) Hypothetical+++, where Ig removed from list** | **Gene** |
| IPI:IPI00001516.1 | Isoform Long of Protocadherin alpha C2 precursor | PCDHAC2 |
| IPI:IPI00829810.1 | Uncharacterized protein ENSP00000375027 | |
| IPI:IPI00854624.1 | Uncharacterized protein ENSP00000375043 | |
| IPI:IPI00854745.1 | Uncharacterized protein ENSP00000375019 | |

**Table 4H-l Hypothetical proteins found in neurologic Post Treatment Lyme Disease**

| **IPI** | |
|---|---|
| IPI00166766 | IPI00852979 |
| IPI00443799 | IPI00374914 |
| IPI00554474 | IPI00003269 |
| IPI00455667 | IPI00749328 |
| IPI00414294 | IPI00644752 |
| IPI00166465 | IPI00749338 |
| IPI00328872 | IPI00794307 |
| IPI00181279 | IPI00847409 |
| IPI00644840 | IPI00411480 |
| IPI00848252 | IPI00739387 |
| IPI00847373 | IPI00855747 |
| IPI00186004 | IPI00384971 |
| IPI00395010 | IPI00061520 |
| IPI00400986 | IPI00006556 |
| IPI00795992 | |

**Table 4H-c**

| **Hypothetical proteins detected in Chronic fatigue** |
|---|
| **IPI** |
| IPI00003269 |
| IPI00006556 |
| IPI00061520 |
| IPI00166766 |
| IPI00168255 |
| IPI00176210 |
| IPI00328872 |
| IPI00374223 |
| IPI00374914 |
| IPI00395010 |
| IPI00396025 |
| IPI00398586 |
| IPI00400986 |
| IPI00413868 |
| IPI00414294 |
| IPI00418966 |
| IPI00419675 |
| IPI00443799 |
| IPI00455667 |
| IPI00550862 |
| IPI00554474 |
| IPI00644752 |
| IPI00749328 |
| IPI00789889 |
| IPI00807406 |
| IPI00852979 |

## Claims

1. A method for diagnosing multiple sclerosis in a patient, the method comprising:
analyzing a biological sample isolated from the patient for expression of a panel of biomarkers including 14-3-3 epsilon protein, to determine if 14-3-3 epsilon protein is identified in the biological sample, wherein identification of 14-3-3 epsilon protein in the biological sample indicates a positive diagnosis of multiple sclerosis in the patient.

2. The method of claim 1 for diagnosing multiple sclerosis in a patient, the method comprising: analyzing a biological sample isolated from the patient for expression of a panel of biomarkers, wherein the panel of biomarkers is set forth in any one of Tables 1m, 3m, 4H-m or Table 2m, to determine if at least two of the biomarkers listed in any one of Tables 1m, 3m, 4H-m or Table 2m, one of which being 14-3-3 epsilon protein, are identified in the biological sample, wherein identification of at least two of the biomarkers in the biological sample indicates a positive diagnosis of multiple sclerosis in the patient.

3. The method of claim 1 for diagnosing multiple sclerosis in a patient, the method comprising: analyzing a biological sample isolated from the patient for expression of a panel of biomarkers, wherein the panel of biomarkers is set forth in any one of Tables 1m, 3m or 4H-m, to determine if at least two of the biomarkers listed in any one of Tables 1m, 3m or 4H-m, one of which being 14-3-3 epsilon protein, are identified in the biological sample, wherein identification of the presence of at least two of the biomarkers in the biological sample indicates a positive diagnosis of multiple sclerosis in the patient.

4. The method of claim 1 for diagnosing multiple sclerosis in a patient, the method comprising: analyzing a biological sample isolated from the patient for expression of a panel of biomarkers, wherein the panel of biomarkers is set forth in Table 1m, to determine if at least two of the biomarkers listed in Table 1m, one of which being 14-3-3 epsilon protein, are identified in the biological sample, wherein identification of the presence of at least two of the biomarkers in the biological sample indicates a positive diagnosis of multiple sclerosis in the patient.

5. The method of any one of claims 1 to 4, wherein the biological sample is cerebrospinal fluid, blood or a component thereof, tissue or tissue-related fluids, urine, or saliva.

## Patentansprüche

1. Verfahren zur Diagnose von multipler Sklerose bei einem Patienten, wobei das Verfahren Analysieren einer dem Patienten entnommenen biologischen Probe auf Expression einer Gruppe von Biomarkern, einschließlich 14-3-3-epsilon-Protein, zur Bestimmung, ob 14-3-3-epsilon-Protein in der biologischen Probe identifiziert wird, umfasst, wobei eine Identifizierung von 14-3-3-epsilon-Protein in der biologischen Probe eine positive Diagnose von multipler Sklerose beim Patienten anzeigt.

2. Verfahren nach Anspruch 1 zur Diagnose von multipler Sklerose bei einem Patienten, wobei das Verfahren Analysieren einer dem Patienten entnommenen biologischen Probe auf Expression einer Gruppe von Biomarkern, wobei die Gruppe von Biomarkern in einer der Tabellen 1m, 3m, 4H-m oder Tabelle 2m angegeben ist, zur Bestimmung, ob wenigstens zwei der in einer der Tabellen 1m, 3m, 4H-m oder Tabelle 2m aufgeführten Biomarker, von denen einer 14-3-3-epsilon-Protein ist, in der biologischen Probe identifiziert werden, umfasst, wobei eine Identifizierung von wenigstens zwei der Biomarker in der biologischen Probe eine positive Diagnose von multipler Sklerose beim Patienten anzeigt.

3. Verfahren nach Anspruch 1 zur Diagnose von multipler Sklerose bei einem Patienten, wobei das Verfahren Analysieren einer dem Patienten entnommenen biologischen Probe auf Expression einer Gruppe von Biomarkern, wobei die Gruppe von Biomarkern in einer der Tabellen 1m, 3m oder 4H-m angegeben ist, zur Bestimmung, ob wenigstens zwei der in einer der Tabellen 1m, 3m oder 4H-m aufgeführten Biomarker, von denen einer 14-3-3-epsilon-Protein ist, in der biologischen Probe identifiziert werden, umfasst, wobei eine Identifizierung des Vorliegens von wenigstens zwei der Biomarker in der biologischen Probe eine positive Diagnose von multipler Sklerose beim Patienten anzeigt.

4. Verfahren nach Anspruch 1 zur Diagnose von multipler Sklerose bei einem Patienten, wobei das Verfahren Analysieren einer dem Patienten entnommenen biologischen Probe auf Expression einer Gruppe von Biomarkern, wobei die Gruppe von Biomarkern in Tabelle 1m angegeben ist, zur Bestimmung, ob wenigstens zwei der in Tabelle 1m aufgeführten Biomarker, von denen einer 14-3-3-epsilon-Protein ist, in der biologischen Probe identifiziert werden, umfasst, wobei eine Identifizierung des Vorliegens von wenigstens zwei der Biomarker in der biologischen Probe eine positive Diagnose von multipler Sklerose beim Patienten anzeigt.

5. Verfahren nach einem der Ansprüche 1 bis 4, wobei es sich bei der biologischen Probe um Liquor, Blut oder einen Bestandteil davon, Gewebe oder gewebebezogene Flüssigkeiten, Urin oder Speichel handelt.

## Revendications

1. Procédé de diagnostic de la sclérose en plaques chez un patient, le procédé comprenant : l'analyse d'un échantillon biologique isolé à partir du patient pour l'expression d'un panel de biomarqueurs comprenant la protéine 14-3-3 epsilon, pour déterminer si la protéine 14-3-3 epsilon est identifiée dans l'échantillon biologique, dans lequel l'identification de la protéine 14-3-3 epsilon dans l'échantillon biologique indique un diagnostic positif de sclérose en plaques chez le patient.

2. Procédé de la revendication 1 pour diagnostiquer la sclérose en plaques chez un patient, le procédé comprenant : l'analyse d'un échantillon biologique isolé à partir du patient pour l'expression d'un panel de biomarqueurs, le panel de biomarqueurs étant décrit dans l'un quelconque des tableaux 1m, 3m, 4H-m ou le tableau 2m, pour déterminer si au moins deux des biomarqueurs répertoriés dans l'un quelconque des tableaux 1m, 3m, 4H-m ou le tableau 2m, dont l'un est la protéine 14-3-3 epsilon, sont identifiés dans l'échantillon biologique, dans lequel l'identification d'au moins deux des biomarqueurs dans l'échantillon biologique indique un diagnostic positif de sclérose en plaques chez le patient.

3. Procédé de la revendication 1 pour diagnostiquer la sclérose en plaques chez un patient, le procédé comprenant : l'analyse d'un échantillon biologique isolé à partir du patient pour l'expression d'un panel de biomarqueurs, le panel de biomarqueurs étant décrit dans l'un quelconque des tableaux 1m, 3m ou 4H-m, pour déterminer si au moins deux des biomarqueurs répertoriés dans l'un quelconque des tableaux 1m, 3m ou 4H-m, dont l'un est la protéine 14-3-3 epsilon, sont identifiés dans l'échantillon biologique, dans lequel l'identification de la présence d'au moins deux des biomarqueurs dans l'échantillon biologique indique un diagnostic positif de sclérose en plaques chez le patient.

4. Procédé de la revendication 1 pour diagnostiquer la sclérose en plaques chez un patient, le procédé comprenant : l'analyse d'un échantillon biologique isolé à partir du patient pour l'expression d'un panel de biomarqueurs, le panel de biomarqueurs étant décrit dans le tableau 1m, pour déterminer si au moins deux des biomarqueurs répertoriés dans le tableau 1m, dont l'un est la protéine 14-3-3 epsilon, sont identifiés dans l'échantillon biologique, dans lequel l'identification de la présence d'au moins deux des biomarqueurs dans l'échantillon biologique indique un diagnostic positif de sclérose en plaques chez le patient.

5. Procédé de l'une quelconque des revendications 1 à 4, dans lequel l'échantillon biologique est du liquide céphalo-rachidien, du sang ou un composant de celui-ci, un tissu ou des fluides associés à un tissu, de l'urine ou de la salive.
